# EUROPEAN PATENT SPECIFICATION

(11) **EP 2 670 742 B1**
(45) Date of publication and mention of the grant of the patent: **29.07.2015**
(21) Application number: 12705207.4
(22) Date of filing: 03.02.2012
(51) Int. Cl.: C07D 301/12, B01J 39/04, B01J 47/02

(54) **A PROCESS FOR REMOVING IRON IONS FROM AN ORGANIC STREAM**
EIN VERFAHREN ZUR ENTFERNUNG VON EISENIONEN AUS EINEM ORGANISCHEN STROM
UN PROCÉDÉ POUR L'ÉLIMINATION DES IONS DE FER CONTENUS DANS UN COURANT ORGANIQUE

(30) Priority: 04.02.2011 US 201161439664 P
(43) Date of publication of application: 11.12.2013
(73) Proprietor: Dow Global Technologies LLC, Midland, MI 48641 (US)
(72) Inventor: CARLBERG, Philip J., Lake Jackson Texas 77566 (US); CRAMPTON, Hannah L., Lake Jackson Texas 77566 (US); GOLTZ, Harlan R., Midland Michigan 48642 (US); LONGORIA, Joe J., Houston Texas 77025 (US)
(74) Representative: Beck Greener
(86) International application number: PCT/US2012/023812
(87) International publication number: WO 2012/106620

(56) References cited:
- EP-A1- 2 072 532
- EP-A2- 0 186 895
- WO-A1-2004/048353
- US-B1- 6 740 763

## Description

### Field of Disclosure

Embodiments of the present disclosure are directed to a process for removing iron ions; more specifically, embodiments are directed to a process for removing iron ions from an organic stream used to produce an oxirane.

### Background

Oxiranes are chemicals that are useful in a variety of end use applications. Epichlorohydrin, for example, is an oxirane that can be used to make epoxy resins. One process for producing oxiranes includes reacting an olefin, a peroxide solution, a catalyst, and a solvent mixture with an alcohol and one or more non-reactive co-solvent(s). For example, an olefin such as allyl chloride can react with a hydrogen peroxide solution to form epichlorohydrin.

Various by-products can form during the process for producing oxiranes. By-products can increase the rate at which the catalyst fouls and/or cause operability problems in the process such as restricting flow and/or plugging. For example, hydrogen peroxide can contain stabilizers that can react with iron ions that have been dissolved by corrosive olefins contacting ferrous vessels and piping. When the corrosive olefins contact the stabilizers a by-product such as a solid precipitate can form. As such, minimizing the formation of by-products can help minimize problems associated with the formation of by-products during the process for producing oxiranes.

### Summary

One or more embodiments of the present disclosure include a process for removing iron ions from an organic stream. For the various embodiments, the process includes providing an organic stream having iron ions; providing an ion exchange resin; removing the iron ions from the organic stream by contacting the organic stream with the ion exchange resin to provide the organic stream with an iron ion concentration of less than one weight percent (wt%) based on a total weight of the organic stream; and contacting the organic stream having the iron ion concentration of less than 1 wt% with a peroxide solution including a stabilizer.

One or more embodiments of the present disclosure also include producing an oxirane product. For the various embodiments, preparing the oxirane product includes providing an organic stream including allyl chloride and iron ions; removing the iron ions from the organic stream with an ion exchange resin by contacting the organic stream with the ion exchange resin to provide the organic stream with an iron ion concentration of less than 1 wt% based on the total weight of the organic stream; and contacting the organic stream having the iron ion concentration of less than 1 wt% with a peroxide solution including a stabilizer.

One or more embodiments of the present disclosure also include a system for producing an oxirane. For the various embodiments, the system includes a first pipe for transporting an organic stream that includes allyl chloride and iron ions; an exchange vessel holding a solid support with active sites that remove iron ions from the organic stream to provide the organic stream with an iron ion concentration of less than 1 wt% based on the total weight of the organic stream, wherein the exchange vessel is connected to the first pipe; a second pipe connected to an outlet of the exchange vessel for transporting the organic stream with the iron ion concentration of less than 1 wt% from the exchange vessel; a third pipe for transporting a peroxide solution including a stabilizer; and a reaction vessel for reacting the organic stream having the iron ion concentration of less than 1 wt% and the peroxide compound to form the oxirane, wherein the second pipe and the third pipe are connected to the reaction vessel.

The above summary of the present disclosure is not intended to describe each disclosed embodiment or every implementation of the present disclosure. The description that follows more particularly exemplifies illustrative embodiments. In several places throughout the application, guidance is provided though lists of examples, which examples can be used in various combinations. In each instance, the recited list serves only as a representative group and should not be interpreted as an exclusive list.

### Brief Description of the Drawings

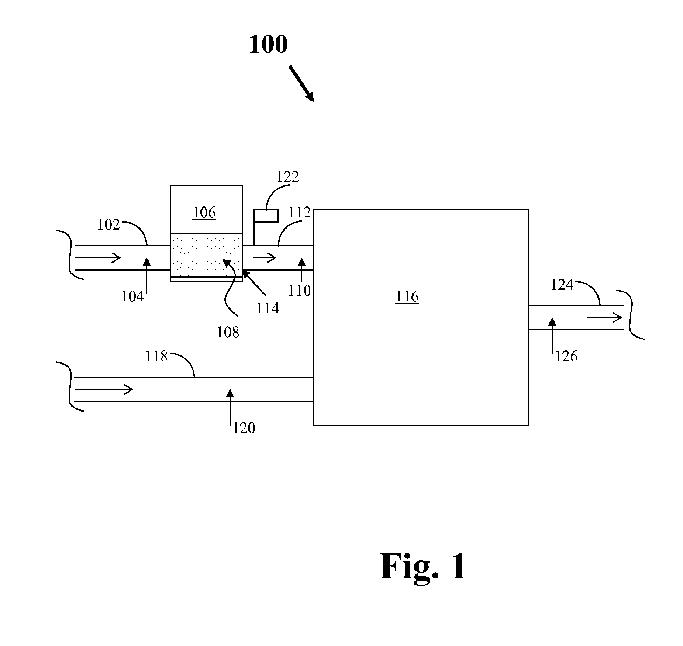

Figure 1 illustrates a system according to an embodiment of the present disclosure.

### Detailed Description

Embodiments of the present disclosure are directed to a process for removing iron ions from an organic stream used in a process to form an oxirane and a system for producing the oxirane with the organic stream. For one or more embodiments, the oxirane is produced by reacting an olefin with a peroxide solution, where such a reaction may also be referred to as an "epoxidation reaction." The iron ions are removed from the organic stream prior to contacting the organic stream with the peroxide solution. For one or more embodiments, the iron ions can be removed by contacting the organic stream with an ion exchange resin.

As discussed herein, one process for producing oxiranes includes reacting the olefin, such as allyl chloride, with the peroxide solution. One issue recognized by the present disclosure is that an organic stream including allyl chloride and more than 50 parts per million (ppm) of water by weight (also referred to herein as "wet allyl chloride") can be corrosive to ferrous metals. When piping of ferrous metal is used to transport wet allyl chloride during the production of the oxirane, the wet allyl chloride can remove iron as ionic iron, i.e., iron ions, from the piping due to this corrosive property. For example, the wet allyl chloride can remove iron as the organic stream comes into contact with ferrous metal based materials (e.g., in ferrous metal piping material and/or vessels).

It is also understood that peroxide solutions can be unstable to the point of decomposing spontaneously to water and oxygen gas. As such, stabilizers are added to the peroxide solution to reduce and/or prevent its decomposition. When the organic stream contacts a peroxide solution that includes such stabilizers, the iron ions and stabilizers can form a precipitate (i.e., a by-product). This precipitate can cause problems during the production of the oxirane, as discussed herein. As such, removing and/or reducing the iron ions in the organic stream before contacting the organic stream with the peroxide solution that includes the stabilizer can reduce an amount of by-products, i.e., the precipitate, formed during the production of the oxirane.

In the following detailed description of the present disclosure, reference is made to an accompanying drawing that forms a part hereof, and in which is shown by way of illustration how one or more embodiments of the disclosure may be practiced. These embodiments are described in sufficient detail to enable those of ordinary skill in the art to practice the embodiments of this disclosure, and it is to be understood that other embodiments may be utilized and that process, electrical, and/or structural changes may be made without departing from the scope of the present disclosure.

The proportion and the relative scale of the elements provided in the figures are intended to illustrate various embodiments of the present invention and are not to be used in a limiting sense.

### Definitions

"Oxirane" refers to a compound in which an oxygen atom is directly attached to two adjacent or non-adjacent carbon atoms of a carbon chain or ring system. Epichlorohydrin, which is formed from an epoxidation reaction of allyl chloride, is an example of an oxirane.

"Solid support" refers to an insoluble matrix (or support structure) formed from an organic polymer or an inorganic material such as silica and other minerals, for example, where the insoluble matrix includes active sites, such as functional groups, for the exchange of ions.

"Stabilizer" refers to a substance that tends to keep a compound, mixture, or solution from changing its form or chemical nature. Examples of such compounds include peroxides. For example, stabilizers can be added to a peroxide solution, such as a hydrogen peroxide solution, to reduce the rate of decomposition.

"Organic stream" refers to a mixture of at least an olefin, such as allyl chloride, and iron ions.

"Peroxide solution" refers to any solution including molecules containing one or more peroxide (-O-O-) functionalities, including organic or inorganic peroxides, peroxide adducts, or peracids.

"Iron ions" refers to the ions of iron including, but not limited to, Fe³⁺, Fe²⁺, and Fe¹⁺, along with combinations of Fe³⁺, Fe²⁺, Fe¹⁺ with other counterions such as Cl⁻, e.g., [FeCl₂]⁺.

"Piping" refers to a system of pipes used to convey fluids (e.g., a liquid) from one location to another location. Such piping can also include in-line components, such as fittings and valves that are used to couple the system of pipes.

As used herein, "a" "an" "the" "at least one" and "one or more" are used interchangeably. The terms "includes" and "comprises" and variations thereof do not have a limiting meaning where these terms appear in the description and claims. Thus, for example, a solvent mixture with an alcohol and a non-reactive co-solvent can be interpreted to mean that the solvent mixture includes one or more alcohol(s) and one or more non-reactive co-solvent(s).

For one or more embodiments, the process includes providing an organic stream having iron ions. For example, the organic stream can be an organic stream including an olefin that can be used to make an oxirane. For one or more embodiments, the olefin is allyl chloride. The organic stream can also include additional olefins. For example, the organic stream can include olefins selected from the group consisting of linear and/or branched acyclic or cyclic aliphatic or aromatic olefins, including those which may contain multiple double bonds. Additional examples of the olefin include, but are not limited to, chloride-butadiene and other linear dialkenes, cyclohexene and other cyclic alkenes and dialkenes, substituted alkenes, such as halogenated alkenes, styrene, divinylbenzene, dicyclopentadiene, other aromatic alkenes and mixtures thereof. Moreover, butenes, pentenes, hexenes, octenes, heptenes, 1-tridecene, mesityl oxide, isoprene, cyclo-octane, cyclohexene or bicyclic compounds such as norbornenes or pinenes may also be used.

As discussed herein, the water in wet allyl chloride can react with the allyl chloride to form hydrochloric acid, thereby becoming corrosive to equipment containing iron, such as piping, in which it comes into contact. The corrosive activity of the hydrochloric acid can dissolve iron as ionic iron (i.e., iron ions) from the piping and incorporate the iron ions into the wet allyl chloride. This corrosive activity helps to form the organic stream, as discussed herein, that contains both the wet allyl chloride and the iron ions. When the iron ions come into contact with stabilizers in the peroxide solution, a precipitate (i.e., a by-product) can form. As discussed herein, reducing by-products such as precipitates can decrease the rate at which the catalyst fouls and/or minimize operability problems in the process such as restricted flow and/or plugging of the process.

Embodiments of the disclosure are discussed with reference to wet allyl chloride becoming corrosive and dissolving iron. However, other olefins may become corrosive by reacting with water and/or other compounds in the organic stream and dissolve iron as well. As such, the present disclosure is not limited to wet allyl chloride.

For one or more embodiments, the process includes providing an ion exchange resin. For one or more embodiments, the ion exchange resin is a strong acid cation exchange resin. Strong acid cation exchange resins contain exchangeable cations, such as hydrogen ions (H⁺). For one or more embodiments, the ion exchange resin includes active sites where ions can be exchanged. For one or more embodiments, the active sites of the ion exchange resin of the present disclosure can be represented by the following formula I:

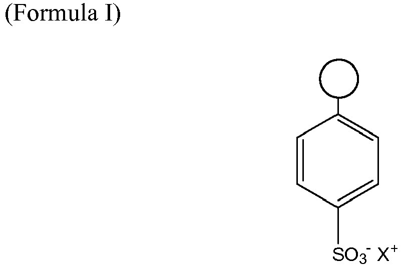

wherein X⁺ is a cation and the circle represents a solid support such as the resin.

For one or more embodiments, the process includes removing the iron ions from the organic stream by contacting the organic stream with the ion exchange resin. Removing the iron ions with the ion exchange resin can be accomplished as the iron ions contact the active site of the ion exchange resin. The ion exchange resin can exchange a cation from the active site with an iron ion in the organic stream. As discussed herein, the ion exchange resin is selected from strong acid cation exchange resins that have an exchangeable cation. In other words, the ion exchange resin can donate an ion to the organic stream and accept an ion from the organic stream. For example, the ion exchange resin can reduce iron ions present in the organic stream by exchanging cations with iron ions in the organic stream.

For one or more embodiments, the iron ions can be removed from the organic stream to provide the organic stream with an iron ion concentration of less than 1 wt%, based on the total weight of the organic stream. In one embodiment, the iron ions are removed from the organic stream to provide the organic stream with an iron ion concentration of less than 1 ppm of the organic stream.

As discussed herein, the iron ions are removed from the organic stream by contacting the organic stream with the ion exchange resin. Contacting the organic stream with the ion exchange resin can be performed in either a batch or continuous mode. For example, the organic stream can be mixed with the ion exchange resin to form a heterogeneous solution. In an additional example, the organic stream can be passed through a fixed-bed reactor that contains the ion exchange resin. For the embodiments, mixing the organic stream with the ion exchange resin in a heterogeneous solution may be performed by known means for mixing, such as, but not limited to, stirring with an agitator or by inducing shear with a mixing element in a tubular reactor or loop reactor. Additionally, using combinations of the reactors and/or of batch and/or continuous modes to contact the organic stream with the ion exchange resin may also be used.

For the embodiments, the amount of the ion exchange resin needed to remove the iron ions from the organic stream will depend on an initial iron ion concentration of the organic stream. The initial iron ion concentration is the concentration of the iron ions prior to contacting the organic stream with the ion exchange resin. The initial iron ion concentration of the organic stream can vary between processes. For example, an amount of exposure (e.g., time) and the conditions of the exposure (e.g, the temperature of the organic stream) at which the organic stream is allowed to contact equipment containing ferrous metal can vary the initial iron ion concentration of the organic stream. Therefore, depending on the initial iron ion concentration of the organic stream the amount of the ion exchange resin will vary. However, enough of the ion exchange resin is used to provide the organic stream with an iron ion concentration that is less than 1 wt%, based on the total weight of the organic stream. In one embodiment, enough of the ion exchange resin is used to provide the organic stream with an iron ion concentration of less than 1 ppm of the organic stream.

For one or more embodiments, the process includes contacting the organic stream having the iron ion concentration of less than 1 wt% with a peroxide solution including a stabilizer. As discussed herein, the organic stream can include the olefin and be used to form the oxirane by reacting the olefin with a peroxide compound in the peroxide solution. For one or more embodiments, the process of the present disclosure can reduce the amount of by-products formed during the epoxidation reaction to form the oxirane. As discussed herein, stabilizers present in the peroxide solution (e.g., a hydrogen peroxide solution) can react with iron ions and form the precipitate. For the embodiments, the organic stream can be used to produce epichlorohydrin by reacting the organic stream that includes allyl chloride and having the iron ion concentration of less than 1 wt% with hydrogen peroxide in the peroxide solution that includes the stabilizer.

For one or more embodiments, the peroxide solution is a hydrogen peroxide solution including a stabilizer, as discussed herein. However, as one skilled in the art would appreciate, other organic and/or inorganic hydroperoxides may be used for the production of the oxirane. Examples of other hydroperoxides that may be used include, but are not limited to, tert-butyl hydroperoxide, ethylbenzene hydroperoxide, acetyl peroxide, benzoyl peroxide, methyl ethyl ketone peroxide, cumene peroxide and combinations thereof.

Available sources of the hydrogen peroxide solution can be produced by the hydrolysis of a persulphuric acid and, more commonly, the successive hydrogenation and oxidation of a substituted alkylanthroquinone in a suitable solvent system. Both methods produce a hydrogen peroxide solution that can contain high levels of impurities such as solids and transition metal ions that are introduced during the production of the hydrogen peroxide solution. Hydrogen peroxide solutions with even trace levels of impurities tend to decompose during storage and/or use. Therefore, stabilizers are added to the hydrogen peroxide solution to reduce and/or prevent decomposition. Examples of stabilizers include, but are not limited to, organic phosphates, phosphoric acid, nitric acid, stannates, or mixtures thereof.

As discussed herein, the iron ions in the organic stream can react with stabilizers (e.g., organic phosphates) and form by-products such as iron phosphate precipitates. Removing the iron ions from the organic stream prior to contacting the organic stream with the hydrogen peroxide solution can help minimize the amount of by-products formed between the iron ions and stabilizers. By-products are solids which can foul the catalyst or restrict flow through the system. For the embodiments of the present disclosure, reducing the by-product formation can also minimize production costs by reducing the need for measures to remove the by-product solids.

For one or more embodiments, the process includes maintaining the iron ion concentration of the organic stream to less than 1 wt%, based on the total weight of the organic stream, preferably to less than 1 ppm of the organic stream before contacting the organic stream with the peroxide solution including the stabilizer. For example, a distance and/or exposure of the organic stream between contacting the organic stream with the ion exchange resin and contacting the organic stream with the peroxide solution can be minimized. Additionally, the equipment used to transport or hold the organic stream having the iron ion concentration of less than one part per million to contact the peroxide solution can be of a non-ferrous materials (e.g., non-ferrous metal materials and/or non-metal materials), discussed more herein.

For one or more embodiments, the process further includes monitoring the iron ion concentration of the organic stream after removing the iron ions from the organic stream with the ion exchange resin. The amount of iron ions the ion exchange resin removes from the organic stream can decrease over time. As such, monitoring the iron ion concentration of the organic stream after contacting the ion exchange resin (e.g., downstream of the ion exchange resin) can help determine when the ion exchange resin is no longer removing the iron ions from the organic stream to at least below 1 wt%, based on the total weight of the organic stream.

Monitoring the iron ion concentration can include positioning a monitoring device after contacting the organic stream with the ion exchange resin. Additionally, monitoring can include periodically taking samples of the organic stream to determine the iron ion concentration. Furthermore, monitoring can include visually determining when the precipitate begins to form between the iron ions and the stabilizers in the peroxide solution.

For one or more embodiments, the process further includes regenerating the ion exchange resin. Ion exchange resins can have a total capacity, which is a total number of chemical equivalents available for exchange per some unit weight or unit volume of the ion exchange resin. Depending on the application, it may be advantageous to regenerate the ion exchange resin at various percentages of the total capacity. For example, the ion exchange resin can be regenerated when the ion exchange resin has reached 20 % of the total capacity, 75 % of the total capacity, or 100 % of the total capacity. As discussed herein, the percent of the total capacity at which the ion exchange resin is regenerated can vary between applications. As such, the ion exchange resin can be regenerated within a range of from greater than zero (0) % of the total capacity to 100 % of the total capacity.

For one or more embodiments, regenerating the ion exchange resin can include treating the ion exchange resin with an acid (e.g., an acid solution). Examples of acids include, but are not limited to, sulfuric acid, hydrochloric acid, and combinations thereof. The ion exchange resin can be treated with a regeneration solution including the acid. The acid solution can have an acid concentration within a range of from 2 to 20 wt%, based on a total weight of the acid solution. The regeneration can be performed within a temperature range of from 25 degrees Celsius (°C) to 80 °C.

For one or more embodiments, a volume of acid is used to generate a majority of the active sites of the ion exchange resin. For example, the majority of the active sites regenerated can be 70 % or greater and preferably 80 % or greater, based on a total number of active sites. As one skilled in the art can appreciate, the volume of acid used can depend on various factors and change between different processes. For example, one factor can include an amount of ion exchange resin being regenerated. For one or more embodiments, a flow rate of the acid solution is 2 bed volumes per hour. After regeneration the acid is rinsed off of the ion exchange resin with deionized water.

For one or more embodiments, the process can further include reacting the organic stream including the olefin and having the iron ion concentration of less than 1 wt% with the peroxide solution to form the oxirane. As discussed herein, the reaction between the olefin and the peroxide solution is referred to as the epoxidation reaction. The epoxidation reaction can occur in the presence of a catalyst, and the solvent mixture with the alcohol and the non-reactive co-solvent. For one or more embodiments, the oxirane is epichlorohydrin.

The catalyst used in the epoxidation reaction can be selected from heterogeneous catalysts which comprise a porous oxide material such as zeolite. As appreciated, zeolites are solid containing silicas which have microporous crystalline ordered channels with a cage structure and pore openings. Along with microporous zeolites, mesoporous and macroporous zeolite type catalysts can also be used. For the embodiments, the catalyst is preferably selected from titanium-silicalites generally known as TS-1 having a MFI structure. It is also possible to use titanium-silicalites with a MEL or intermediate MFI/MEL structure and titanium-silicalites from beta zeolites containing titanium and having a BEA structure. Other titanium containing zeolite catalysts generally known as TS-2, TS-3, ZSM-48 and ZMS-12 can also be used.

For the embodiments, a portion or all of the titanium in the zeolite catalyst can be replaced by, but not limited to, boron, aluminum, iron, gallium, vanadium, zirconium, chromium, niobium or a mixture of two or more thereof. Additional examples of zeolites containing titanium, vanadium, chromium, niobium, and zirconium include, but are not limited to, BEA, MOR, TON, MTW, FER, CHA, ERI, RHO, GIS, BOG, NON, EMT, HEU, KFI, FAU, DDR, MTT, RUT, RTH, LTL, MAX, GME, NES, OFF, SGT, EUO, MFS, MWW and ITQ-4. It is also possible to use titanium-containing zeolites having the UTD-1, CIT-1 or CIT-5 structure in the process of the present invention. Furthermore, other heterogeneous and homogeneous catalysts may be used. Examples include, but are not limited to, soluble metal catalysts such as ligand-bound rhenium, tungsten, and manganese, along with the heterogenized forms of these.

For the embodiments, the catalyst can be used in a range of from 0.1 wt% to 30 wt%, more preferably in a range of from 0.1 wt% to 15 wt%, and still more preferably in a range of from 0.1 wt% to 5 wt%, based on the total weight of the reaction mixture, which includes the olefin, peroxide solution, catalyst, and solvent mixture with the alcohol and the non-reactive co-solvent.

Catalysts used in epoxidation reactions will eventually deactivate. The formation of by-products such as precipitates can increase the rate of deactivation by plugging the pores of the catalyst. As provided herein, the process for removing iron ions from the organic stream can help minimize the amount of by-products formed. Minimizing the by-products can reduce the rate at which the pores of the catalyst become plugged. Reducing the rate at which the pores of the catalyst become plugged can increase the lifetime of the catalyst as compared to a process using an organic stream where iron ions have not been removed. For the embodiments, increasing the lifetime of the catalyst and reducing the frequency at which the catalyst needs to be separated and regenerated can reduce the cost and time associated with epoxidation reactions.

As discussed herein, the epoxidation reaction is carried out in the presence of the solvent mixture with the alcohol and non-reactive co-solvent. The alcohol can be selected from protic solvents. For example, alcohols, such as methanol, ethanol, isopropyl alcohol, tert-butyl alcohol and cyclohexanol, can be used. For the embodiments, the solvent is preferably methanol. Mixtures of the various alcohols may also be used.

For the embodiments, the amount of the protic solvent in the reaction mixture can be within a range of from 0.5 wt% to 90 wt%, more preferably within a range of from 0.5 wt% to 50 wt%, and still more preferably within a range of from 1 wt% to 10 wt%, based on the total weight of the reaction mixture.

For the embodiments, the non-reactive co-solvent can be selected from non-water soluble solvents that include, but are not limited to, linear and cyclic alkanes of C₃-C₁₈, halogenated hydrocarbons, deactivated aromatics, amides, solvents containing nitriles, alcohols, and halogenated alcohols or mixtures thereof. Examples of the non-reactive co-solvent include, but are not limited to, carbon tetrachloride, propyl chloride, chloroform, dichloromethane, dichloroethane, hexane, octane, decalin, perfluorodecalin, mono or poly-chlorinated benzenes, mono- or poly-brominated benzenes, acetophenone, benzonitrile, acetonitrile, tritolyl phosphate, trichlorotrifluoroethane, trichloroethanol, trifluoroethanol or mixtures thereof. For the embodiments, the non-reactive co-solvent is preferably 1,2-dichlorobenzene.

The non-reactive co-solvent can be used within in a range of from 5 wt% to 70 wt%, more preferably within a range of from 10 wt% to 65 wt%, and still more preferably within a range of from 25 wt% to 50 wt%, based on the total weight of the reaction mixture.

For the embodiments, the epoxidation reaction is carried out at a predetermined reaction temperature. For the embodiments, the predetermined reaction temperature can be within a range of from 0 °C to 100 °C, more preferably within a range of from 10 °C to 80 °C, and still more preferably within a range of from 40 °C to 60 °C. In addition, the predetermined reaction temperature can remain at a constant temperature during the epoxidation reaction. For the embodiments, the epoxidation reaction is performed at 1 standard atmosphere, but other pressures may be used. Additionally, the pressure may be modified during the epoxidation reaction.

The reaction mixture is formed when the organic stream, which includes the olefin, contacts the peroxide solution in the present of the catalyst and the solvent mixture with the alcohol and the reactive co-solvent. The reaction mixture includes the olefin, the peroxide solution, the catalyst, the alcohol, and the non-reactive co-solvent. The reaction mixture can further include additional reagents.

For the embodiments, a substantial portion of the resulting oxirane will form in a liquid organic phase that can include the olefin, the non-reactive co-solvent, and a portion of the by-products. However, a portion of the resulting oxirane may reside in a liquid aqueous phase that can include hydrogen peroxide, the alcohol, and a portion of the by-products.

The present disclosure further describes an oxirane product formed by the processes described herein. The oxirane product is formed by reacting the organic stream, including allyl chloride, with a peroxide solution. As discussed herein, the organic stream can contain iron ions that can react with stabilizers present in the peroxide solution. The iron ions can be removed from the organic stream with an ion exchange resin, as discussed herein, by contacting the organic stream with the ion exchange resin to provide the organic stream with an iron ion concentration of less than 1 wt% based on the total weight of the organic stream. The organic stream having the iron ion concentration of less than 1 wt% is contacted with a peroxide solution including a stabilizer to form the oxirane. As discussed here, the peroxide solution can be a hydrogen peroxide solution and the oxirane can be epichlorohydrin.

Embodiments of the present disclosure further provide a system for producing the oxirane. Figure 1 illustrates a system 100 according to an embodiment of the present disclosure. The system 100 includes a first pipe 102 for transporting an organic stream 104 including allyl chloride and iron ions, where the organic stream 104 has the initial iron ion concentration. The first pipe 102 transports the organic stream 104 to an exchange vessel 106 that holds a solid support 108 with active sites that can remove iron ions from the organic stream 104 to provide the organic stream with an iron ion concentration of less than 1 wt% based on the total weight of the organic stream. Organic stream 110 leaving the exchange vessel 106 has the iron ion concentration of less than 1 wt%, based on the total weight of the organic stream 110. For one or more embodiments, the solid support 108 with active sites is an ion exchange resin, and in particular, a strong acid cation exchange resin. As discussed herein, removing the iron ions with the ion exchange resin can be carried out in either a continuous, a semi-continuous, or in a batch process. The exchange vessel 106 can be selected from, but not limited to, one or more continuous stirred tank reactors, tubular reactors, fixed-bed reactors, and combinations thereof. For one embodiment, the exchange vessel 106 is a fixed-bed reactor.

For one or more embodiments, the system 100 includes a second pipe 112 connected to an outlet 114 of the exchange vessel 106. The second pipe 112 can transport the organic stream 110 having the iron ion concentration of less than 1 wt%, based on the total weight of the organic stream 110, from the exchange vessel 106 to a reaction vessel 116.

For one or more embodiments, the system 100 can include a third pipe 118 connected to the reaction vessel 116. The third pipe 118 can transport a peroxide solution 120 including a stabilizer. For one or more embodiments, the peroxide solution 120 is a hydrogen peroxide solution. The organic stream 110 including allyl chloride and having the iron ion concentration of less than 1 wt%, based on the total weight of organic stream 110, can react with the peroxide solution 120 in the reaction vessel 116 to form the oxirane.

As seen in Figure 1, the organic stream 110 and the peroxide solution 120 are introduced separately into the reaction vessel 116 via the second pipe 112 and the third pipe 118, respectively. In one or more embodiments, the organic stream 110 and the peroxide solution 120 can be combined prior to entering the reaction vessel 116.

As discussed herein, the epoxidation reaction can be carried out in either a continuous, a semi-continuous, or in a batch process. The epoxidation reaction can also be carried out in at least one batch reactor or at least one continuous reactor, or in a combination of these. For example, the reactor vessel 116 may be selected from, but not limited to, one or more continuous stirred tank reactors, tubular reactors and combinations thereof. In one embodiment, the reaction vessel 116 is a continuous stirred tank reactor.

For one or more embodiments, the second pipe 112 that transports the organic stream 110 from the exchange vessel 106 to the reaction vessel 116 can be of a non-ferrous metal material. Having the second pipe 112 constructed from non-ferrous metal materials can maintain the iron ion concentration of the organic stream 110 to less than 1 wt%, based on the total weight of the organic stream 110, prior to contacting the organic stream 110 with the peroxide solution 120 to form the oxirane.

As discussed herein, the amount of iron ions the ion exchange resin removes from the organic stream can decrease over time. The iron ion concentration of the organic stream 110 can be monitored to determine when the iron ion concentration of the organic stream 110 is approaching or above 1 wt%, based on the total weight of the organic stream 110. As such, the system can further include a monitoring device 122 positioned at the outlet 114 of the exchange vessel 106 or downstream from the exchange vessel 106 to measure the iron ion concentration in the organic stream 110 prior to contacting the organic stream 110 with the peroxide solution 120 in the reaction vessel 116.

The system can further include a fourth pipe 124 that can remove a reaction effluent 126 from the reaction vessel 116. The fourth pipe 124 can carry the reaction effluent 126 downstream for further processing that can include, but is not limited to, decantation, extraction, and/or distillation.

### EXAMPLES

The following examples are given to illustrate the scope of this invention.

### Materials

Organic stream, allyl chloride (99.4% purity) including 3.5 wt % of iron ions. Allyl chloride available from The Dow Chemical Company.

Ion exchange resin, AMBERLITE™ IR120 H (CAS# 9002-23-7), available from The Dow Chemical Company.

Ion exchange resin, DOWEX™ DR-2030 (CAS#69011-20-7), available from The Dow Chemical Company.

### Test Methods

### X-Ray Fluorescent (XRF) Test

Samples were transferred to an XRF sample cup and analyzed using Uniquant on the Philips PW-2400 wavelength dispersive x-ray fluorescence spectrometer. UniQuant analyzes for more than 60 elements including all common metals (aluminum, zinc, vanadium, nickel, iron, manganese, cobalt, molybdenum, copper, titanium, calcium, magnesium, chromium, sodium, and potassium). UniQuant also analyzes for non-metals such as P, S and Cl. The accepted detection limit for most elements in this method is 0.1 wt% and the accuracy for this method is generally better than ±30% and can be dependent on the assumed matrix.

### Inductively Coupled Plasma Spectrometry (ICP) Test

Samples were weighed into quartz crucibles and approximately 2 milliliters of sulfuric acid was added. The samples were ashed in a muffle furnace at 550 °C until all of the organic components were removed. The ash was then dissolved in aqua regia (nitro-hydrochloric acid), the samples were made up to weight with deionized water and they were analyzed using an ICP spectrometer.

### Examples 1-8

### Example 1

AMBERLITE™ IR120 H (ion exchange resin) (2.10 grams (g)) was placed into a 20-mililiter vial. The organic stream (21.12 g) was added to the vial containing the ion exchange resin. The contents of the vial were agitated with a magnetic stirrer for 16 hours. After the 16 hours, the agitation was stopped and a sample of the organic stream was collected with a syringe. The collected sample was filtered using a 0.45 micron syringe filter. The sample was analyzed by XRF to determine the iron ion concentration in the organic stream. The results are illustrated in Table I.

### Example 2

The procedure in Example 1 was repeated, but with the following changes: use 3.94 g of the ion exchange resin and 20.01 g of the organic stream.

### Example 3

The procedure in Example 1 was repeated, but with the following changes: use 6.23 g of the ion exchange resin and 20.42 g of the organic stream.

### Example 4

The procedure in Example 1 was repeated, but with the following changes: use 1.12 g of the ion exchange resin and 19.98 g of the organic stream.

### Example 5

DOWEX™ DR-2030 (ion exchange resin) (0.18 g) was placed into a 20-mililiter vial. The organic stream (20.37 g) was added to the vial containing the ion exchange resin. The contents of the vial were agitated with a magnetic stirrer for 16 hours. After the 16 hours, the agitation was stopped and a sample of the organic stream was collected with a syringe. The collected sample was filtered using a 0.45 micron syringe filter. The sample was analyzed by ICP to determine the iron ion concentration in the organic stream. The results are illustrated in Table I.

### Example 6

The procedure in Example 5 was repeated, but with the following changes: use 0.32 g of the ion exchange resin, and 20.54 g of the organic stream.

### Example 7

The procedure in Example 5 was repeated, but with the following changes: use 0.38 g of the ion exchange resin, and 20.51 g of the organic stream.

### Example 8

The procedure in Example 5 was repeated, but with the following changes: use 0.52 g of the ion exchange resin, and 21.02 g of the organic stream.

**Table I**

| **Example** | **Ion Exchange Resin (g)** | **Allyl Chloride (g)** | **Fe** |
|---|---|---|---|
| Example 1 | 2.10 | 21.12 | ND |
| Example 2 | 2.94 | 20.01 | ND |
| Example 3 | 6.23 | 20.42 | ND |
| Example 4 | 1.12 | 19.98 | ND |
| Example 5 | 0.18 | 20.37 | <1 ppm |
| Example 6 | 0.32 | 20.54 | <1 ppm |
| Example 7 | 0.38 | 20.51 | <1 ppm |
| Example 8 | 0.52 | 21.02 | <1 ppm |

Table 1 is a summary of Examples 1 through 8. In the table, "ND" stands for "none detected" by XRF, which means the iron ion concentration is less than 0.1 wt% of the organic stream. The organic stream prior to removing the iron ions had an initial iron ion concentration of 3.5 wt%, based on the total weight of the organic stream. As seen in Table I, each example reduces the iron ion concentration of the organic stream to below 1 wt%. Additionally, Examples 5 through 8 reduce the iron ion concentration of the organic stream to below one part per million of the organic stream.

## Claims

1. A system for producing an oxirane, comprising:
a first pipe for transporting an organic stream including allyl chloride and iron ions;
an exchange vessel holding a solid support with active sites that remove iron ions from the organic stream to provide the organic stream with an iron ion concentration of less than one weight percent based on the total weight of the organic stream, wherein the exchange vessel is connected to the first pipe;
a second pipe connected to an outlet of the exchange vessel for transporting the organic stream with the iron ion concentration of less than one weight percent from the exchange vessel;
a third pipe for transporting a peroxide solution including a stabilizer; and
a reaction vessel for reacting the organic stream having the iron ion concentration of less than one weight percent and the peroxide compound to form the oxirane, wherein the second pipe and the third pipe are connected to the reaction vessel.

2. The system of claim 1, wherein the solid support is a strong acid

3. The system of any one of claims 1-2, wherein the second pipe is of a non-ferrous metal material.

4. The system of any one of claims 1-3, wherein the exchange vessel is arranged in a fixed-bed configuration holding the solid support.

5. The system of any one of claims 1-4, wherein the exchange vessel is of a non-ferrous metal material.

6. A process for removing iron ions from an organic stream, the process comprising:
providing an organic stream having iron ions;
providing an ion exchange resin;
removing the iron ions from the organic stream by contacting the organic stream with the ion exchange resin to provide the organic stream with an iron ion concentration of less than one weight percent based on the total weight of the organic stream; and
contacting the organic stream having the iron ion concentration of less than one weight percent with a peroxide solution including a stabilizer.

7. The process of claim 6, wherein contacting the organic stream with the ion exchange resin includes contacting the organic stream with active sites of the ion exchange resin, wherein the active sites are represented by the following formula I: 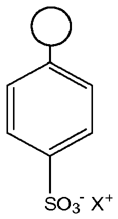 wherein X⁺ is a cation and the circle represents a solid support.

8. The process of any one of claims 6-7, wherein the iron ion concentration of the organic stream after removing the iron ions is less than one part per million of the organic stream.

9. The process of any one of claims 6-8, wherein the peroxide solution is a hydrogen peroxide solution that contains the stabilizer.

10. The process of any one of claims 6-9, further including regenerating the ion exchange resin.

11. The process of claim 10, wherein regenerating the ion exchange resin includes treating the ion exchange resin with an acid.

12. The process of claim 11, wherein the acid is selected from the group consisting of sulfuric acid, hydrochloric acid, and combinations thereof

13. The process of any one of claims 6-12, further including reacting the organic stream including an olefin with the peroxide solution in the presence of a catalyst and a solvent mixture with an alcohol and a non-reactive co-solvent to form an oxirane.

14. The process of claim 13, wherein the oxirane is epichlorohydrin.

## Patentansprüche

1. Ein System zum Herstellen eines Oxirans, beinhaltend:
eine erste Leitung zum Transportieren eines organischen Stroms, einschließlich Allylchlorid und Eisenionen;
einen Austauschbehälter, der einen festen Träger mit aktiven Zentren hält, die Eisenionen aus dem organischen Strom entfernen, um den organischen Strom mit einer Eisenionenkonzentration von weniger als einem Gewichtsprozent, bezogen auf das Gesamtgewicht des organischen Stroms, zu versehen, wobei der Austauschbehälter mit der ersten Leitung verbunden ist;
eine zweite Leitung, die mit einem Auslass des Austauschbehälters zum Transportieren des organischen Stroms mit der Eisenionenkonzentration von weniger als einem Gewichtsprozent vom Austauschbehälter verbunden ist;
eine dritte Leitung zum Transportieren einer Peroxidlösung, einschließlich eines Stabilisators; und
einen Reaktionsbehälter, um den organischen Strom mit der Eisenionenkonzentration von weniger als einem Gewichtsprozent und die Peroxidverbindung zur Reaktion zu bringen, um das Oxiran zu bilden, wobei die zweite Leitung und die dritte Leitung mit dem Reaktionsbehälter verbunden sind.

2. System gemäß Anspruch 1, wobei der feste Träger eine starke Säure ist.

3. System gemäß einem der Ansprüche 1-2, wobei die zweite Leitung aus einem Nichteisenmetallmaterial besteht.

4. System gemäß einem der Ansprüche 1-3, wobei der Austauschbehälter in einer Festbettkonfiguration angeordnet ist, die den festen Träger hält.

5. System gemäß einem der Ansprüche 1-4, wobei der Austauschbehälter aus einem Nichteisenmetallmaterial besteht.

6. Ein Verfahren zum Entfernen von Eisenionen aus einem organischen Strom, wobei das Verfahren beinhaltet:
Bereitstellen eines organischen Stroms, der Eisenionen aufweist;
Bereitstellen eines lonenaustauschharzes;
Entfernen der Eisenionen aus dem organischen Strom durch In-Kontakt-Bringen des organischen Stroms mit dem lonenaustauschharz, um den organischen Strom mit einer Eisenionenkonzentration von weniger als einem Gewichtsprozent, bezogen auf das Gesamtgewicht des organischen Stroms, zu versehen; und
In-Kontakt-Bringen des organischen Stroms mit der Eisenionenkonzentration von weniger als einem Gewichtsprozent mit einer Peroxidlösung, einschließlich eines Stabilisators.

7. Verfahren gemäß Anspruch 6, wobei das In-Kontakt-Bringen des organischen Stroms mit dem lonenaustauschharz das In-Kontakt-Bringen des organischen Stroms mit aktiven Zentren des lonenaustauschharzes umfasst, wobei die aktiven Zentren durch die folgende Formel I dargestellt werden: 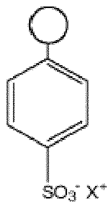 wobei X⁺ ein Kation ist und der Kreis einen festen Träger darstellt.

8. Verfahren gemäß einem der Ansprüche 6-7, wobei die Eisenionenkonzentration des organischen Stroms nach dem Entfernen der Eisenionen weniger als ein Teil pro Million des organischen Stroms beträgt.

9. Verfahren gemäß einem der Ansprüche 6-8, wobei die Peroxidlösung eine Wasserstoffperoxidlösung ist, die den Stabilisator enthält.

10. Verfahren gemäß einem der Ansprüche 6-9, das ferner das Regenerieren des lonenaustauschharzes umfasst.

11. Verfahren gemäß Anspruch 10, wobei das Regenerieren des lonenaustauschharzes das Behandeln des lonenaustauschharzes mit einer Säure umfasst.

12. Verfahren gemäß Anspruch 11, wobei die Säure aus der Gruppe, bestehend aus Schwefelsäure, Salzsäure und Kombinationen davon ausgewählt ist.

13. Verfahren gemäß einem der Ansprüche 6-12, das ferner das Zur-Reaktion-Bringen des ein Olefin umfassenden organischen Stroms mit der Peroxidlösung in Gegenwart eines Katalysators und eines Lösemittelgemisches mit einem Alkohol und einem nichtreaktiven Co-Lösemittel umfasst, um ein Oxiran zu bilden.

14. Verfahren gemäß Anspruch 13, wobei das Oxiran Epichlorhydrin ist.

## Revendications

1. Un système pour produire un oxirane, comprenant :
un premier tuyau pour transporter un flux organique incluant du chlorure d'allyle et des ions fer ;
une cuve d'échange contenant un support solide avec des sites actifs qui retirent des ions fer du flux organique afin de fournir au flux organique une concentration en ions fer de moins d'un pour cent en poids rapporté au poids total du flux organique, la cuve d'échange étant raccordée au premier tuyau ;
un deuxième tuyau raccordé à une sortie de la cuve d'échange pour transporter le flux organique avec la concentration en ions fer de moins d'un pour cent en poids depuis la cuve d'échange ;
un troisième tuyau pour transporter une solution de peroxyde incluant un stabilisant ; et
une cuve de réaction pour faire réagir le flux organique ayant la concentration en ions fer de moins d'un pour cent en poids et le composé peroxyde afin de former l'oxirane, le deuxième tuyau et le troisième tuyau étant raccordés à la cuve de réaction.

2. Le système de la revendication 1, dans lequel le support solide est un acide fort.

3. Le système de n'importe laquelle des revendications 1 à 2, dans lequel le deuxième tuyau est en un matériau de métal non ferreux.

4. Le système de n'importe laquelle des revendications 1 à 3, dans lequel la cuve d'échange est arrangée dans une configuration à lit fixe contenant le support solide.

5. Le système de n'importe laquelle des revendications 1 à 4, dans lequel la cuve d'échange est en un matériau de métal non ferreux.

6. Un procédé pour retirer des ions fer d'un flux organique, le procédé comprenant :
le fait de fournir un flux organique ayant des ions fer ;
le fait de fournir une résine échangeuse d'ions ;
le fait de retirer les ions fer du flux organique en mettant en contact le flux organique avec la résine échangeuse d'ions afin de fournir au flux organique une concentration en ions fer de moins d'un pour cent en poids rapporté au poids total du flux organique ; et
le fait de mettre en contact le flux organique ayant la concentration en ions fer de moins d'un pour cent en poids avec une solution de peroxyde incluant un stabilisant.

7. Le procédé de la revendication 6, dans lequel le fait de mettre en contact le flux organique avec la résine échangeuse d'ions inclut le fait de mettre en contact le flux organique avec des sites actifs de la résine échangeuse d'ions, les sites actifs étant représentés par la formule I suivante : 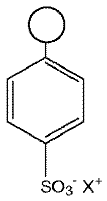 dans laquelle X⁺ est un cation et le cercle représente un support solide.

8. Le procédé de n'importe laquelle des revendications 6 à 7, dans lequel la concentration en ions fer du flux organique après avoir retiré les ions fer est inférieure à une partie par million du flux organique.

9. Le procédé de n'importe laquelle des revendications 6 à 8, dans lequel la solution de peroxyde est une solution de peroxyde d'hydrogène qui contient le stabilisant.

10. Le procédé de n'importe laquelle des revendications 6 à 9, incluant en outre le fait de régénérer la résine échangeuse d'ions.

11. Le procédé de la revendication 10, dans lequel le fait de régénérer la résine échangeuse d'ions inclut le fait de traiter la résine échangeuse d'ions avec un acide.

12. Le procédé de la revendication 11, dans lequel l'acide est sélectionné dans le groupe constitué d'acide sulfurique, d'acide hydrochlorique, et de combinaisons de ceux-ci.

13. Le procédé de n'importe laquelle des revendications 6 à 12, incluant en outre le fait de faire réagir le flux organique incluant une oléfine avec la solution de peroxyde en présence d'un catalyseur et un mélange de solvant avec un alcool et un cosolvant non réactif afin de former un oxirane.

14. Le procédé de la revendication 13, dans lequel l'oxirane est une épichlorhydrine.
